# EUROPEAN PATENT SPECIFICATION

(11) **EP 1 515 741 B1**
(45) Date of publication and mention of the grant of the patent: **23.03.2011**
(21) Application number: 03759947.9
(22) Date of filing: 12.06.2003
(51) Int. Cl.: A61K 38/16, A61K 38/39, A61K 31/715, A61K 31/731, A61K 31/732, A61K 31/736, A61K 31/723, A61P 3/00

(54) **Use of collagen hydrolysate to lower the viscosity of a composition comprising guar gum**
Verwendung von Kollagen-Hydrolysat zur Reduktion der Viskosität einer Guarkernmehl enthaltenden Zusammensetzung
L'utilisation de hydrolysat de collagène pour réduire la viscosité d'une composition comprenant la gomme de guar

(30) Priority: 13.06.2002 GB 0213612
(43) Date of publication of application: 23.03.2005
(73) Proprietor: Nestec S.A., 1800 Vevey (CH)
(72) Inventor: AURIOU, Nicolas, CH-3012 Bern (CH); BEER, Michael, CH-3098 Köniz (CH)
(74) Representative: Lock, Graham James
(86) International application number: PCT/EP2003/006194
(87) International publication number: WO 2003/105882

(56) References cited:
- EP-A- 0 323 510
- EP-A- 0 855 181
- WO-A-01/43758
- GB-A- 2 021 948

## Description

The present invention concerns a viscous soluble fiber composition. The invention also relates to a method for lowering the viscosity of a viscous soluble fiber composition and to a composition for use in preventing or treating diabetes, hypercholesterolemia, obesity, and/or metabolic syndrome in a mammal.

The importance of dietary fiber in the daily diet has been emphasized over the last several years through the recognition of its effects in the prevention and/or treatment of such diseases as hyperlipidemia, coronary heart disease, arteriosclerosis, colon diverticulum, colon cancer, heart diseases, hypercholesterolemia, obesity, hypertension, diabetes or metabolic syndrome. Metabolic syndrome can be characterized by at least one of the following abnormalities: abdominal obesity, hyperlipidemia, hypercholesterolemia, high blood pressure and high blood glucose level. Recent studies have shown that more than 20 % of the US adult population has the metabolic syndrome.

Soluble fibers act in promoting bowel action. The bulk-forming property of such fiber also contributes to fill the stomach, which has a satiety effect. Soluble fibers delay the emptying of the stomach and slow the transit of chyme in the upper gastrointestinal tract, resulting in slower absorption rates and altered hormonal responses to the absorbed nutrients. It has been proposed that viscous soluble fibers, such as konjac gum or oat beta glucan, may reduce the need for insulin, improve glycemia, reduce serum (LDL) cholesterol, slow the appearance of glucose in the blood and inhibit bile acid absorption in the ileum.

A potential difficulty in adopting viscous soluble fiber in a composition is the viscosity that is generally associated with levels considered to be therapeutic in the treatment of certain diseases, such as diabetes or metabolic syndrome. In particular, the high viscosity may render problematic the preparation of a composition for oral and enteral administration, especially for patients requiring tube-feeding. Therefore there is a need for a viscous soluble fiber composition, which is non-viscous, i.e. sufficiently low viscous to be enterally and/or orally administered.

Present inventors have surprisingly found that proteins, such as wheat protein, egg protein, collagen protein, whey protein, casein protein, gluten, pea protein and/or hydrolysates thereof, have the ability to lower the viscosity of viscous soluble fiber compositions, such as konjac gum and/or xanthan gum and/or guar gum and/or beta glucan and/or pectin compositions. According to the invention nutritional compositions comprising a high level of high viscous soluble fiber may be formulated, which are convenient to administer, yet without necessarily increasing the glucidic value of such compositions. After ingestion, the viscosity of the soluble fibers comprised in the compositions according to the invention may re-increase because of the digestion and the consecutive hydrolysis of the viscosity-lowering protein.

In a first aspect of the invention there is provided use, as defined in claim 1, of collagen hydrolysate to lower the viscosity of a composition comprising guar gum.

In another aspect of the invention there is provided use, as defined in claim 2, of a composition comprising hydrolysed collagen and guar gum for the manufacture of a medicament for prevention or treatment of metabolic syndrome and/or diabetes and/or for lowering blood cholesterol level.

In a further aspect of the invention there is provided a composition, as defined in claim 5, for use in prevention or treatment of metabolic syndrome, and/or diabetes and/or for lowering blood cholesterol level.

The invention can be used in a method of treating and/or preventing diabetes and/or metabolic syndrome and/or of lowering blood cholesterol level in a mammal in need of such a treatment comprising administering to said mammal an effective amount of a composition for use of the invention.

As described herein, a method for providing viscous soluble fibers to a mammal in need of such fibers comprises administering to said mammal a composition for use of the invention.

The term "soluble fiber" as used herein refers to non-starch polysaccharides characterized as being soluble by using the official method of the Association of Official Analytical Chemists (Prosky et al, 1988; J. Assoc. Off. Anal. Chem, 70, 5, 1017), e.g. water soluble fibers, e.g. water soluble at room temperature.

As used herein, the term "viscous fiber" refers to fibers which are able to increase the viscosity of the contents of the stomach and the small intestine and to slow gastric emptying rates. Typically, compositions comprising 1 % by weight of such viscous fibers have a viscosity higher than about 500 mPa.s (500 centipoises) at room temperature, e.g. temperature from 20°C to 25°C, e.g. more than about 1000 mPa.s, or more than about 1500 mPa.s. In one aspect of the invention, such viscous fibers have a molecular weight between about 20'000 and about 5 millions Da, e.g. between about 100'000 and about 2 millions Da.

As used herein, the term "dietary fiber" refers to non-starch polysaccharides non hydrolyzed, or only partially hydrolyzed, by the human digestive enzymes. These polysaccharides may contain beta 1,4 or beta 1,6 bonds.

For the purpose of the present invention, the term "compositions of the invention" are as defined in the appended claims, and encompasses compositions for oral or enteral administration. Such compositions may be non-viscous, e.g. less viscous than same compositions in which the viscosity-lowering protein is absent, and/or hydrolyzed, e.g. moderately or extensively hydrolyzed. In another aspect of the invention, the compositions according to the invention, e.g. the non-viscous compositions of the invention, may be defined by a viscosity less than about 500 mPa.s, e.g. less than about 250 or less than about 150 mPa.s, e.g. less than about 100 mPa.s or less than about 60 mPa.s. In one aspect the compositions of the invention may have a viscosity of between about 60 and about 500 mPa.s at room temperature. It has to be understood that such viscosities are measured with a Brookfield LVDV II viscosimeter at room temperature, e.g. temperature from 20° to 25°C, and for a spindle and a shear rate in agreement with the product thickness. For example, viscosities lower than 60 mPa.s are measured with a LVDV II viscosimeter having a spindle of 1 and at 100 rotations per minute (rpm).

The compositions described herein have the advantageous characteristic that the viscosity may be modulated, for example reversed, delayed, or re-increased. As used herein, "modulated viscosity" refers to the viscosity of a soluble viscous fiber composition that has been modulated, i.e. lowered, or totally or partially re-increased, e.g. by moderately or extensively hydrolyzing the viscosity-lowering protein. As used herein, "delayed viscosity" refers to the viscosity of compositions which has been re-increased, e.g. by moderately or extensively hydrolyzing the viscosity-lowering protein, or by denaturing the viscosity-lowering protein. As used herein, "moderately hydrolyzed protein" refers to protein which has a molecular weight between about 1 and about 20 kDa, e.g. between about 5 and about 15 kDa. As used herein, "extensively hydrolyzed protein" refers to protein which has a molecular weight lower than about 0.5 kDa, e.g. lower than 0.2 kDa or lower than about 0.1 kDa. As used herein, "denatured protein" refers to protein having no further viscosity lowering activity in the composition to which it is added or in which it is present. The hydrolysis or denaturation of the viscosity-lowering protein may occur in vivo, i.e. after ingestion of the compositions of the invention, e.g. non-viscous compositions, converting the compositions of the invention from low- or non-viscous to high-viscous compositions, resulting in e.g. a slower glucose absorption and/or in a lower or slower glucose and insulin response to carbohydrate loads. In one aspect of the invention, there is provided a composition comprising viscous soluble fiber(s) and viscosity-lowering protein(s) wherein the viscosity-lowering effect of the protein, e.g. the viscosity of the composition, is reversible.

In one embodiment of the invention, compositions for use of the invention may consist exclusively or essentially of viscous soluble fiber, e.g. viscous soluble dietary fiber, and viscosity-lowering protein. Alternatively, the compositions of the invention may comprise other nutritional and/or pharmaceutical components in addition to viscous soluble fiber and viscosity-lowering protein.

In one embodiment of the invention, the compositions of the invention do not comprise hydrolyzed, e.g. extensively hydrolyzed, soluble viscous fiber. As used herein, "hydrolyzed, e.g. extensively hydrolyzed, soluble viscous fiber" refers to non-viscous soluble fiber, e.g. having a molecular weight between about 300 and about 20'000 Da, e.g. between about 500 and about 10'000 Da. In one aspect of the invention, the compositions of the invention comprise native guar gum. Optionally the compositions according to the invention may comprise moderately hydrolyzed soluble viscous fiber, e.g. moderately hydrolyzed guar gum. As used herein, "moderately hydrolyzed viscous fiber" refers to fibers having a molecular weight between about 20'000 and about 5 millions Da, e.g. between about 100'000 and about 2 millions Da.

The fiber of the compositions of the invention may consist essentially or exclusively of viscous soluble fibers, e.g. viscous soluble dietary fibers, optionally in the form of native viscous soluble fibers, or in the form of moderately hydrolyzed viscous soluble fiber, or mixtures thereof. In a further embodiment of the invention, the compositions of the invention do not comprise non-viscous soluble fiber. In yet a further embodiment, the compositions of the invention do not comprise inulin.

The compositions of the invention comprise one viscous soluble fiber, which is guar gum.

Xanthan gum, is referred to herein. It is the extracellular polysaccharide from *Xanthomonas campestris* and some related microorganisms, is produced on a nutritive medium comprising glucose, a nitrogen source and minerals. It is used in the food industry as a stabilizer or thickener. It is also known to interact synergistically with other hydrocolloids like guar gum. Xanthan gum is commercially available from Satiaxane CX2QD, Degussa Texturant System, France.

Konjac glucomannan is referred to herein. It is also called konjac mannan or konjac gum, has the highest viscosity of any dietary fiber known. Konjac gum has a high capacity for water absorption and can swell to about 200 times its original volume. In food industry, konjac gum is often added to starch because their interaction leads to a considerable increase in the viscosity and to the formation of gel, which can be maintained during cooking. Konjac gum is also known to interact synergistically with hydrocolloids such as kappa-carrageenan, xanthan gum or locust bean gum to form heat-stable gels. Konjac gum may also gel by itself if heated after alkali addition. According to the present invention, konjac gum may be extracted from the *Amorphopallus* species, more particularly from *A. riviera* (often referred to as *A. konjac), A. oncophyllus, A. variais, A. bulbifera* and *A. blumeli,* more preferably from *A. riviera,* and even more preferably from *A. riviera C. Koch.* In particular konjac gum is produced from the root of the plant *Amorphopallus,* and more preferably from the tuber thereof. Konjac flour may be prepared by drying, pulverizing and winnowing the plant. Several techniques are known in the art for separating konjac glucomannan from konjac flour. As used herein the term "konjac gum" encompasses konjac glucomannan and konjac flour. The konjac gum is commercially available from Propol RS, Shimizu, Japan.

Guar Gum is obtained from the seed endosperms of the leguminous plant *Cyamopsis tetragonolobus* (Linné) Taub. and consists mainly of a high molecular weight hydrocolloidal polysaccharide, composed of galactose and mannose units combined through glycoside linkages. Guar Gum is used in the food industry as a stabilizer or thickener. It is also known to interact synergistically with other hydrocolloids like xanthan gum. Guar gum is commercially available from Polygum 75, Polygal AG, Switzerland (high molecular weight type) and from Lygomme 3, Degussa Texturant Systems, France (low molecular weight type). As used herein, the term "guar gum" encompasses high molecular weight type and low molecular weight type.

Mixed-linkage (1→3)(1→4)-β-D-glucan (beta-glucan) is referred to herein. It is the main endospermic cell wall polysaccharide of oats and barley, but also present, in lesser amounts, in rye and wheat. The subaleurone and subembryo layers of dehulled oat are particularly rich in beta-glucan, whereas in the inner starchy endosperm concentrations are low. Beta-glucan can therefore be enriched in bran fractions by 3-4 times. Beta-glucan is commercially available from Megazyme International, Ireland.

Pectin is referred to herein. It is purified carbohydrate product obtained from the dilute acid extract of the inner portion of the rind of, citrus fruits or from apple pomace. It consists mainly of partially methoxylated polygalacturonic acids. It is commercially available from Riedel de Haen AG, Germany.

Details of the active ingredients of the compositions of the invention may be obtained from the relevant manufacturers.

The relative proportion of the active ingredients of the compositions of the invention will, of course, vary considerably depending on the particular type of composition concerned, e.g. whether it is a liquid or solid form, or whether it is provided in nutritional form. All indicated proportions and relative weight ranges described below are accordingly to be understood as being indicative of preferred or individually inventive teaching only and not limiting the invention in its broadest aspect.

According to the present invention, the term "soluble viscous fiber" is referred to as fiber concentrate or not pure fiber source. Fiber concentrates may be guar gum. Fiber concentrate of the invention may comprise more than 90 % by weight of fiber, based on the total dry weight of the fiber concentrate, preferably more than 95% by weight. Not pure fiber source may comprise more than 80% by weight of fiber.

Furthermore the compositions described herein may comprise insoluble fibers, such as cellulose, hull fibers from legumes and grains, such as pea hull fiber, oat hull fiber, barley hull fiber, soy hull fiber. In one embodiment of the invention, the ratio of soluble viscous fiber to insoluble fiber may be from 0.1 to 10, preferably from 0.2 to 5.

The term "viscosity-lowering protein" as used herein refers to proteins or peptides which when added to a viscous soluble fiber composition may lower the viscosity of that composition. These proteins may have a molecular weight lower than about 35 kDa, more preferably lower than about 30 kDa, even more preferably from about 0.2 to about 30 kDa, and even more preferably from about 1 to about 25 kDa. These values refer to an approximate molecular weight range with values falling above and below the given range. The viscosity-lowering proteins may be in the form of native protein or in form of hydrolysate.

Examples of "viscosity-lowering protein" include wheat protein, soy protein, pea protein, egg protein, collagen, whey protein, casein, gluten, muscle proteins from fish and animals, fibrillar proteins, silk proteins, e.g. keratin, sericin or fibroin, and mixtures thereof. Accordingly, in one composition described herein, it comprises wheat protein, egg protein, collagen, whey protein, casein, soy protein, pea protein, fibrillar proteins, silk proteins, e.g. keratin, sericin or fibroin, hydrolysates thereof or mixtures thereof, preferably wheat protein, egg protein, collagen, fibrillar proteins, silk proteins, e.g. keratin, sericin or fibroin hydrolysates thereof or mixtures thereof. In another composition described herein, the composition comprises whey protein, casein, soy protein, pea protein and/or hydrolysate thereof, with the proviso that said compositions do not comprise inulin.

Described herein is a composition wherein the composition does not comprise phytoestrogen or a xanthine derivative. In one composition, there is provided soy protein and/or hydrolysate thereof, with the proviso that said composition does not comprise phytoestrogen or a xanthine derivative.

In an embodiment of the invention, the compositions of the invention consist essentially of, or exclusively of, guar gum and collagen hydrolysate.

The amount of viscous soluble fiber incorporated into the compositions of the invention may depend on the nature and/or the molecular weight of the viscous fiber, the nature and the amount of the viscosity-lowering protein, the form of the compositions of the invention, e.g. a powder or a composition ready-for-consumption, -e.g. ready-to-drink composition or instant drink-, or on the final viscosity to be reached, e.g. the type of product in which the compositions will be incorporated. Typically, the more viscous the viscous soluble fiber is, the less it amounts in the compositions.

Accordingly, suitable amounts of viscous soluble fiber comprised in ready-for-consumption compositions according to the invention are in the range of up to about 40% by weight, e.g. up to about 20% by weight, or up to about 15% by weight, for example from about 0.05 to about 10% by weight, e.g. from about 0.5 or 1 to about 2.5, 5 or 10%, e.g, about 1% by weight, based on the total weight of the ready-for-consumption composition.

The compositions of the invention in powder form may comprise viscous soluble fiber in an amount of about 0.5 to about 60% by weight, more preferably in an amount of about 4 to about 40% by weight, more preferably in an amount of about 10 to about 30% by weight and even more preferably in an amount of about 8 to about 20% by weight, based on the total weight of the powder composition.

The suitable amount of viscosity lowering-proteins incorporated into the compositions of the invention may depend on the molecular weight and/or on the nature of the protein, for example on its three-dimensional structure, e.g. globular, fibrillar, denaturated structure, its charge, or its pHi.

The amount of viscosity-lowering protein incorporated into the ready-for-consumption compositions of the invention may vary from about 1 to about 60% by weight, preferably from about 2 to about 20 % by weight, more preferably from about 3 to about 10% by weight, even more preferably from about 4 to about 8 % by weight, based on the total weight of the composition.

In one embodiment of the invention, the compositions of the invention in powder form comprise an amount of viscosity-lowering protein from about 0.05 to 90% by weight, preferably from about 0.5 to about 80% by weight, more preferably from about 20 to about 60% by weight of the composition, based on the total weight of the composition.

In a further aspect of the invention, the compositions of the invention may comprise the viscosity lowering-protein in an amount sufficient to provide a non-viscous composition, e.g. for oral or enteral administration, e.g. a composition having a viscosity lower than 500 mPa.s.

In another embodiment of the invention, the weight ratio of viscous soluble fiber : viscosity lowering-protein in the compositions of the invention, in powder or in ready for consumption form, may be from about 0.01:1 to about 20:1, preferably from about 0.05:1 to about 10:1, more preferably from about 0.1:1 to about 1:1, and most preferably from about 0.1:1 to about 0.4:1.

In one aspect of the invention, the total amount of viscous soluble fiber and viscosity lowering-protein in the compositions of the invention, in powder or in ready for consumption form, may be from about 1 to about 65% by weight, more preferably from about 1 to about 30% by weight, even more preferably from 2 to 15% by weight and most preferably from about 5 to about 15% by weight, based on the total weight of the composition.

In one embodiment of the invention, the compositions of the invention may comprise other proteinaceous material in addition to the viscosity lowering-protein. "Proteinaceous material" as used herein refers to any form of protein, peptide, free amino acid, mixtures of amino acids, and combinations thereof.

In a further embodiment of the invention, the compositions of the invention may comprise, in addition to soluble viscous fiber and viscosity lowering-protein, other nutritional components like fat, carbohydrate, minerals and vitamins, for example, among others, calcium, magnesium, iron, zinc, phosphorus, vitamin D and/or vitamin K.

In another embodiment of the invention, the carbohydrate content of the compositions according to the invention, including viscous soluble fiber, may be less than about 60 en%, preferably less than about 55 en%, more preferably less than about 30 en%, most preferably less than about 20 en%, based on the total caloric content of the composition.

In yet another embodiment of the invention, the compositions according to the invention may not comprise starch-derived carbohydrates, such as maltodextrin, modified starch, amylose starch, topioca starch, corn starch, or mixtures thereof.

In another embodiment of the invention, the compositions according to the invention may not comprise saccharose.

In a further embodiment of the invention, the compositions according to the invention may comprise fructose and/or galactose.

Further components of the compositions according to the invention may include any bioactive compounds or extracts which are known to have health benefits, especially for lowering blood cholesterol level in mammals, such as statins, bile acid sequestrants, nicotinic acid or fibric acids, and/or for treating diabetes, such as sulfonylureas, biguanides, alpha-glucosidase inhibitors, thiazolidinediones, meglitinides or D-phenylalamine. For example, the composition of the invention may be provided in the form of a kit for separate, sequential or simultaneous administration in conjunction with such medicines for lowering blood glucose and/or blood cholesterol level. These medicines may conveniently be formulated together with viscous soluble fiber, such as e.g. konjac gum, xanthan gum, beta-glucan or guar gum, and a viscosity-lowering protein in standard pharmaceutical dosage forms.

In one aspect of the present invention, the compositions according to the invention can readily be incorporated into pharmaceutical or nutritional formulations, typically nutraceuticals, dietary supplements, functional food and beverage products.

In a further aspect of the invention, the compositions of the invention may be used as a medicament. Accordingly the compositions of the invention may be administered in pharmaceutical form or as a dietary supplement, preferably in combination with at least one pharmaceutically or nutritionally acceptable carrier.

In a yet further aspect of the invention, there is provided a medicament, nutritional or pharmaceutical formulation, for example dietary supplement, comprising the composition of the invention. The medicament, nutritional or pharmaceutical composition of the invention may optionally comprise pharmaceutical acceptable carriers. Further, according to the invention there is provided a combined pharmaceutical preparation for simultaneous, separate or sequential use for the treatment or prevention of diabetes comprising soluble viscous fiber and viscosity-lowering protein and one or more drug(s) for lowering blood glucose and/or one or more drug(s) for lowering blood cholesterol level.

Pharmaceutical compositions and dietary supplements may be provided in the form of soft gel, sachets, powders, syrups, liquid suspensions, emulsions and solutions in convenient dosage forms. In soft capsules the active ingredients are preferably dissolved or suspended in suitable liquids, such as fatty oils, paraffin oil or liquid polyethylene glycols. Optionally stabilisers may be added.

Oral pharmaceutical or dietary supplement forms may be made by conventional compounding procedures known in the pharmaceutical art, that is, by mixing the active substances together with edible pharmaceutically acceptable solid or liquid carriers and/or excipients, e.g. fillers such as cellulose, lactose, sucrose, mannitol, sorbitol, and calcium phosphates and binders, such as starch, gelatin, tragacanth, methylcellulose and/or polyvinylpyrrolidone (PVP). Optional additives include lubricants and flow conditioners, e.g. silicic acid, silicon dioxide, talc, stearic acid, magnesium/calcium stearates, polyethylene glycol (PEG) diluents, disintegrating agents, e.g. starch, carboxymethyl starch, cross-linked PVP, agar, alginic acid and alginates, colouring agents, flavouring agents, and melting agents. Dyes or pigments may be added to the tablets or dragée coatings, for example for identification purposes or to indicate different doses of active ingredient.

Optionally, the compositions according to the invention may be nutritionally complete, i.e. may include vitamins, minerals, trace elements as well as nitrogen, carbohydrate and fatty acid sources so that they may be used as the sole source of nutrition supplying essentially all the required daily amounts of vitamins, minerals, carbohydrates, fatty acids, proteins and the like. Accordingly, the compositions of the invention may be provided in the form of a nutritionally balanced complete meal, e.g. suited for oral or tube feeding.

Alternatively, the compositions of the invention may be provided as part of a meal, i.e. a nutritional supplement, e.g. in the form of a drink, e.g. a health drink.

It may be desirable to provide the soluble viscous fiber in the form of a low calorie meal replacement or other nutritional product. In this case the meal replacement or other nutritional product is preferably low fat, i.e. less than about 10 en%, or substantially fat-free, i.e. less than about 2.5 en% contributed by fat, such as about 2 en% fat, based on the total caloric content of the composition. Suitably, a single serving of a low calorie meal replacement will have a caloric value of less than about 1000kcal, and preferably between about 200kcal and about 500kcal. Suitable low calorie nutritional product may include soft drink, such as juice, smoothie or soy-based drink, or dispersed in foods of any sort, such as, dairy bars, soups, breakfast cereals, muesli, candies, tabs, cookies, biscuits, crackers, such as a rice crackers, and dairy products, such as milk-shake, yoghurt drink.

The compositions of the invention optionally comprise conventional food additives, such as any of emulsifiers, stabilizers, sweeteners, flavourings, colouring agents, preservatives, chelating agents, osmotic agents, buffers or agents for pH adjustment, acidulants, thickeners, texturisers, and so on.

In a further aspect of the invention, there is provided a use of compositions of the invention as food additive.

The method of decreasing the viscosity of the compositions of the invention may advantageous in the preparation of food products and beverages. For example, the compositions described herein, e.g. low viscous compositions, may be used for preparing a high viscous nutritional product. The compositions described herein may also be used to lower the viscosity, to stabilize and/or to texturize food and beverage products, such as ice creams, candies, chocolates, muesli, cookies, biscuits, crackers, chewing-gums, dairy products, desserts, milk-shakes, yogurt drinks, soups and the like. Optionally the compositions of the invention may be used as a fat substitute in a variety of reduced fat, low fat and fat free foods and beverages, such as cakes, pudding type desserts, butter, peanut butter, cheeses, salad dressings, sauces, margarine, cream cheese and other spreads, snack dips, mayonnaise, sour cream, yogurt, ice cream, frozen desserts, fudge and other confections, and skim milk. The compositions described herein are also useful, for example as a shortening, in baked goods such as cakes, pies, brownies, cookies, breads, noodles, snack items, such as crackers, graham crackers and pretzels, and similar products.

Suitable product formats include solution, ready-for-consumption composition, e.g. ready-to-drink compositions, instant drink, liquid comestibles, like soft drinks, juice, sports drinks, milk drinks, milk-shakes, yogurt drinks or soup. In a further embodiment, the compositions of the present invention may be manufactured and sold in the form of a concentrate, a powder, or granules, e.g. effervescent granules, which are diluted with water or other liquid, such as milk or fruit juice, to yield a ready-for-consumption composition, e.g. ready-to-drink compositions or instant drink.

Enteral administration of the compositions of the invention, and preferably oral administration are intended.

The amount and dosage regimen of the compositions for use of the invention to be administered is determined in the light of various relevant factors including the purpose of administration, the age, sex and body weight of individual subject and the severity of the subject's symptoms.

When the composition for use according to the invention is supplied in the form of a food or beverage, a suitable serving size of the composition may be from about 20 to about 500g, preferably from about 30 to about 300g. If provided in pharmaceutical form, suitable daily doses of the composition of the invention are up to about 250g, preferably 150g, and optimally in the range of about 10 to about 100g. In terms of body weight, an appropriate daily dosage range is about 2 to about 15g composition of the invention per kg. The daily dosage may correspond to a single unit dosage, or may be provided through multiple unit dosages. The exact amounts of the compositions according to the invention may vary between wide limits and may be readily determined in conventional manner.

The compositions for use of the invention, e.g. medical or nutritional formulations comprising the compositions for use of the invention, have the advantageous characteristic of decreased viscosity, thus allowing delivery of an effective amount of viscous soluble fibers. Suitable daily doses of viscous soluble fiber according to the invention are in the range of about 2 mg to about 50 g, preferably in the range of about 25 mg to about 40 g, more preferably in the range of about 50 mg to about 30 g and most preferably in the range of about 2 g to about 20 g.

The compositions for use of the invention may be administered under the supervision of a medical specialist, or may be self-administered.

The compositions for use of the invention may be administered daily, weekly or monthly, or may be administered annually or even for longer periods in order for example to constantly control the glucose or cholesterol blood level of a patient suffering from diabetes or high blood cholesterol level. Suitable daily dosage regimen may include single or multiple servings per day. Optimally, the composition for use of the invention is consumed at least once a day on a regular basis, prior to, i.e. pre- or post-prandial, or during a meal. Preferably, the compositions for use of the invention may be consumed prior to a meal. In one embodiment of the invention, the composition for use of the invention may be taken until for example a blood glucose level of 180 mg/dL or less, 2 hours after meals, has been resumed, or until a blood cholesterol level of 200 mg/dL or less has been obtained, and/or a blood LDL cholesterol level of less than160mg/dL, or less than 130mg/dL has been resumed Since these formulations are safe to consume, subjects with diabetes, metabolic syndrome and/or hypercholesterolemia can continue taking composition according to the invention for as long as required, and preferably until normal blood glucose level, 2 hours after meals, and/or cholesterol level has been resumed.

Pharmaceutical, food or beverage incorporating compositions for use according to the invention can be safely-consumed by anyone, and are especially recommended for anyone perceived to be at risk from hypercholesterolemia, diabetes or metabolic syndrome, such as elderly, smokers and/or anyone suffering from overweight, obesity, heart disease or high blood pressure. The compositions of the invention may be particularly suitable for subjects suffering from metabolic syndrome, blood sugar disorders, arteriosclerosis, hypertension, hypercholesterolemia, hyperlipidemia, diabetes, intestinal or digestive troubles, Crohn's disease, Irritable Bowel Syndrom (IBS), Inflammatory Bowel Disease (IBD), intestinal diverticula, carcinoma of colon. By lowering blood glucose level, the compositions of the invention have also the effect of counteracting the sequelea of heart disease, high blood pressure, stroke, blindness, blood vessel disease, kidney failure, amputation and nerve damage. "High blood pressure" as used herein refers to a blood pressure persistently exceeding 140/90 mmHg (systolic/diasystolic). By lowering blood cholesterol level, the compositions of the invention may also prevent heart diseases, e.g. heart attacks.

The invention can be used for a method of treating and/or preventing metabolic syndrome and/or diabetes and/or hypercholesterolemia, in a mammal, including human, in need of such a treatment, wherein the treatment comprises administering to said mammal an effective amount of a composition according to the invention. As used herein, the term "an effective amount" refers to an amount effective to achieve a desired therapeutic effect, such as treating and/or preventing diabetes and/or metabolic syndrome and/or hypercholesterolemia.

The invention can be used for a method of lowering blood cholesterol level and/or blood glucose in a mammal, including human, in need of such a treatment comprising administering to said mammal an effective amount of a composition according to the invention. Hereinafter the terms "lowering blood glucose" refer to lowering the blood glucose to a level of 180 mg/dL or less 2 hours after meals. As used herein, "lowering blood cholesterol level" refers to a blood cholesterol lowered to a level of less than 240mg/dL, more preferably less than 200 mg/dL, or to a blood LDL cholesterol lowered to less than 190mg/dL, more preferably less than 160 mg/dL, even more preferably less than 130 mg/dL.

The compositions according to the invention may be used in the manufacture of a medicament or nutritional formulation for the prevention or treatment of metabolic syndrome and/or diabetes and/or hypercholesterolemia in mammal, including human.

In a further embodiment of the invention, the compositions according to the invention may be used in the manufacture of a medicament or nutritional formulation for lowering blood glucose and/or blood cholesterol level in mammal.

The compositions for use of the invention may be used in meeting the dietary fiber intake of a mammal.

In one aspect of the invention, the method of treatment as herein described may comprise the combined administration with diabetes medicines, such as sulfonylureas, biguanides, alpha-glucosidase inhibitors, thiazolidinediones, meglitinides or D-phenylalamine, and/or blood cholesterol lowering medicines, such as statins, bile acid sequestrants, nicotinic acid and fibric acids.

A low viscous composition comprising viscous soluble fiber may be produced by admixing guar gum and a viscosity-lowering protein selected from hydrolysed collagen in an aqueous medium under conditions sufficient to form a non-viscous composition, e.g. a composition which has a viscosity lower than the viscosity of the same composition in which the viscosity-lowering protein is absent. Suitable examples of aqueous medium include, but are not limited to, water, milk, water-based beverages, milk-based beverages, carbonated beverage, non-carbonated beverage, beer, wine, soy milk and fruit-based beverage, like orange juice. Such aqueous medium may also include specific buffers, like phosphate buffer.

According to the invention, the ingredients may be added simultaneously or sequentially, i.e. the viscosity-lowering protein followed or preceded by the viscous soluble fiber, to the aqueous medium, as defined above.

In one embodiment of the invention, the ingredients of the compositions of the invention may be added in dry form, i.e. as a dry blend of viscous soluble fiber and viscosity-lowering protein. Such a dry blend may be dispersed into an aqueous medium by stirring or mixing, preferably using cold, hot or boiling aqueous medium, and more preferably using aqueous medium at a temperature of about 5°C to about 35°C. In one aspect of the invention the composition of the invention in form of a drink may be obtained by dispersing the blend, e.g. dry blend, of viscous soluble fiber and viscosity-lowering protein in cold water, e.g. water at a temperature of between about 5 and about 35°C.

In another embodiment of the invention, the dried ingredients of the compositions for use of the invention may be hydrated separately before being mixed, i.e. a dispersion of soluble viscous fiber and/or a dispersion of viscosity-lowering protein may be prepared separately and then mixed. Suitable techniques of hydrating viscous soluble fibers and obtaining such dispersions are known in the art. The conditions chosen, such as temperature or stirring time, for hydrating the viscous soluble fiber will depend in particular on the nature and concentration of the fiber. Such conditions are known in the art. To obtain a hydrated soluble viscous fiber dispersion, the soluble viscous fiber may be added to a cold, hot or boiling aqueous medium, more preferably to an aqueous medium at a temperature from about 70°C to about 80°C, and stirred for one to several hours, for example for 1 hour. Preferably, the concentration of the soluble viscous fiber in the dispersion will vary from about 0.05 to about 15 % by weight, more preferably from about 0.5 to about 5% by weight, based on the total weight of the dispersion. In one embodiment of the invention, the soluble viscous fiber dispersion may be prepared in a phosphate buffer, e.g. at a pH from about 6.7 to about 7.2. Lower pH values may also be used, e.g. a pH from about 3.8 to about 5.7. For example, a konjac gum dispersion may be prepared by adding konjac flour in an amount of from about 0.5 to about 5% by weight, based on the total weight of the dispersion, to a phosphate buffer solution, having a pH from about 6.7 to about 7.2, at room temperature, and by stirring for approximately 5 minutes. The hydrated viscosity-lowering protein dispersion may be prepared by hydrating the dry viscosity-lowering protein in an aqueous medium at room temperature. The concentration of the viscosity-lowering protein in the dispersion may vary from about 0.1 to about 60% by weight, preferably from about 0.5 to about 35%, more preferably from about 1 to about 10% by weight, based on the total weight of the dispersion. For example, collagen hydrolysate dispersion may be prepared by stirring for less than 5 minutes in a cold aqueous medium.

The dried soluble viscous fiber may be added to a viscosity-lowering protein dispersion, e.g. hydrated protein, or the dried viscosity-lowering protein may be added to a soluble viscous fiber dispersion. In either case, the dried component is added to a dispersion at a low temperature, at room temperature or at a high temperature, for example from about 5°C to about 60°C, more preferably from about 15°C to about 25°C.

According to the invention, the concentration of soluble viscous fiber may vary from about 0.05 to about 30% by weight, preferably from about 0.1 to about 10% by weight, more preferably from about 0.5 to about 5% by weight, and even more preferably from about 0.75% to about 2% by weight, based on the total weight of the composition. The concentration of the viscosity-lowering protein may be from about 1 to about 60% by weight, preferably from about 2 to about 20% by weight, more preferably from about 3 to about 10% by weight, even more preferably from about 4 to about 8% by weight, based on the total weight of the composition. In a further step, the composition for use of the invention may be dehydrated to obtain a powder.

It will be appreciated that the suitable method of dispersion will be chosen based on the desired properties and/or uses of the compositions for use of the invention.

According to the invention, the viscosity of the compositions of the invention may be lowered. It is also possible to modulate and/or delay, preferably re-increase, the viscosity of the non-viscous compositions, by modulating and/or delaying the degradation state of the viscosity-lowering protein. The more viscosity-lowering protein is degraded, e.g. denatured or hydrolyzed, the less is its effect on lowering the viscosity, and the more the viscosity re-increases. Suitable methods of denaturizing or hydrolyzing proteins are known in the art. For example, the viscosity-lowering proteins of the compositions of the invention may be denatured or hydrolyzed by proteases, such as pepsin, trypsin, chymotrypsin, exopeptidase or endopeptidase, at a temperature from about 20°C to about 60°C, preferably at about 37°C. Optionally, the viscosity of the compositions, e.g. non-viscous or low-viscous compositions, may be re-increased by heating and simultaneously using an alkaline treatment, e.g. at a pH of about 12, or more preferably by using an acidic treatment, e.g. at a pH of about 4.5, more preferably at a pH below 2. Various degrees of modulation of the viscosity may be obtained by varying the degree of denaturation or hydrolysis, e.g. the hydrolysis conditions, such as for example temperature, incubation period, nature of enzyme and/or pH. A combination of two or more denaturation or hydrolysis methods, e.g. of enzymatic and acid hydrolysis, may also be used. As described herein, the compositions for use of the invention may be incubated in the presence of a protease under conditions and time sufficient to denature or hydrolyze the viscosity-lowering protein to the extent that it has no further viscosity lowering activity, e.g. no longer suppresses the viscosity of the viscous soluble fiber.

A method of denaturizing or hydrolyzing the viscosity-lowering protein(s) is described herein to re-increase or reverse The viscosity on a composition on the invention, which method comprises the addition of means, such as proteases, e.g. pepsin, trypsin, chymotrypsin, exopeptidase or endopeptidase, in amounts sufficient to induce degradation, e.g. denaturation or hydrolysis, of the viscosity-lowering-protein(s), as well as acidic or basic solutions in amounts sufficient to decrease or increase, respectively, the pH of the composition.

In yet a further aspect there is provided a use of means as hereinabove described for denaturizing or hydrolyzing the viscosity-lowering protein(s) to re-increase or reverse the viscosity of a composition of the invention.

The utility of all the compositions of the present invention may be observed in standard clinical tests in, for example, indications as hereinabove described, for example using daily dosages of viscous soluble fiber in the range of about 2 mg, 25 mg, 50 mg or 2 g to about 20, 30, 40 or 50 g, and viscosity lowering protein in dosages sufficient to provide a non-viscous composition of the invention, for a mammal, e.g. adult and in standard animal models. The blood glucose lowering effect or blood cholesterol lowering effect may be observed in standard animal tests and in clinical trials.

One human clinical trial may be affected as follows:
A single blind, placebo controlled, parallel study in 90 subjects may be performed using the composition of the invention, e.g. comprising about 2 mg, 25 mg, 50 mg or 2 g to about 20, 30, 40 or 50 g viscous soluble fiber and viscosity lowering protein in dosages sufficient to provide a non-viscous composition of the invention, to study the effects on metabolic syndrome characteristics. The following parameters may be assessed at baseline and after 3 month of treatment: body weight, weight regain and composition of weight regain, attitude towards eating, appetite profile, oral glucose tolerance (OGT), glucose, insulin, C-peptide, triglyceride (TG), Glycerol, free fatty acids (FFA) and satiety (all subjects).

According to the invention it is possible to effectively ameliorate symptoms and conditions associated with metabolic syndrome, diabetes or high blood cholesterol levels with natural compounds, which do not show any severe side effects. Further, the present methods are well-tolerated and simple to apply.

The invention is further illustrated with reference to the following Examples.

### Examples

Konjac gum is from Propol RS, Shimizu, Japan.

Collagen hydrolysate is from Collagel A, Deutsche Gelatine-Fabriken Stoess & Co GmbH, Germany. High MW Guar type is from Polygum 75, Polygal AG, Switzerland.

Low MW Guar type is from Lygomme 3, Degussa Texturant Systems, France.

Xanthan gum is from Satiaxane CX2QD, Degussa Texturant System, France.

Aspartame is from Searle, USA (Nutrasweet^{™}).

Acesulfam K is from Nutrinova, Germany (Sunett^{™}).

### Example 1: (Comparative Example) Influence of viscosity-lowering protein on a 1% konjac gum composition.

The konjac gum and the viscosity-lowering protein are admixed together and dispersed in water at temperature of about 25°C. A magnetic agitation is used as dispersion tools and the mixing is kept for 2 min. After 1 hour standing time the viscosity is measured.

| Protein | Molecular Weight (kDa) | Protein Content ⁽¹⁾ | Konjac Content ⁽¹⁾ | Protein Solution Viscosity* | Final Solution Viscosity** |
|---|---|---|---|---|---|
| Glycin | 0.1 | 10 | 1 | <10 | >5000 |
| Wheat | <1 | 10 | 1 | <10 | 200 |
| Egg | 3-10 | 10 | 1 | 50 | 250 |
| Collagen | 10-18 | 10 | 1 | 10 | 60 |
| Whey | 10-18 | 10 | 1 | 25 | 500 |
| - | - | - | 1 | - | >5000 |

| | | | | | |
|---|---|---|---|---|---|
| ⁽¹⁾ % by weight, based on the total weight of the composition. * Viscosity (mPa.s) measured with a Brookfield viscometer (LVDV number 1 spindle, 60 rpm). ** Viscosity (mPa.s) measured with a Brookfield viscometer (LVDV number 4 spindle, 60 rpm) after 30 minutes of standing time. | | | | | |

### Example 2: (Comparative Example) Influence of the collagen concentration on the viscosity of a 1% konjac gum composition.

Compositions comprising konjac gum and collagen hydrolysate at different concentrations were prepared as described in Example 1, and their viscosities were measured at 25°C with a Brookfield viscometer using a LVDV number 4 spindle and a shear rate of 60 rpm.

| | | | | | |
|---|---|---|---|---|---|
| Konjac gum ⁽¹⁾ | 1 | 1 | 1 | 1 | 1 |
| Collagen ⁽¹⁾ | 10 | 6.5 | 5 | 3.5 | 0 |
| Viscosity | 60 | 50 | 50 | 90 | >5000 |

| | | | | | |
|---|---|---|---|---|---|
| ⁽¹⁾ % by weight, based on the total weight of the composition. | | | | | |

### Example 3: (Comparative Example) Influence of collagen on the viscosity of konjac gum and/or xanthan gum compositions.

The viscous soluble fiber compositions were prepared as described in Example 1, and their viscosities were measured.

| | | | | | |
|---|---|---|---|---|---|
| Konjac gum ⁽¹⁾ | - | 0.8 | 0.8 | - | 1 |
| Xanthan gum ⁽¹⁾ | 0.2 | 0.2 | 0.2 | 0.2 | - |
| Collagen ⁽¹⁾ | - | - | 5 | 5 | - |
| Viscosity (mPa.s) | 120** | 1500* | 400** | 15** | 7000* |

| | | | | | |
|---|---|---|---|---|---|
| ⁽¹⁾ % by weight, based on the total weight of the composition. * Viscosity measured with a Brookfield viscometer using a LVDV number 4 spindle and a shear rate of 60 rpm. ** Viscosity measured with a Brookfield viscometer using a LVDV number 2 spindle and a shear rate of 60 rpm. | | | | | |

### Example 4: (According to the Invention) viscosity of guar gum compositions.

The compositions were prepared as described in the Example 1. The viscosities were measured with a Brookfield viscometer with LV spindle at 20°C.

### A. Influence of molecular weight and concentration of guar gum on the viscosity.

| | | | | | | | | | |
|---|---|---|---|---|---|---|---|---|---|
| High MW guar gum (g) | 1 | 2 | 3 | 4 | - | - | - | - | - |
| Low MW guar gum (g) | - | - | - | - | 1 | 2 | 3 | 4 | 5 |
| Water (g) | 199 | 198 | 197 | 196 | 199 | 198 | 197 | 196 | 195 |
| Viscosity (mPa.s) | 330 | 4100 | 28000 | 170000 | 75 | 1020 | 4500 | 15000 | 58000 |

### B. Viscosity lowering effect of collagen on guar gum compositions.

| | | | | | | |
|---|---|---|---|---|---|---|
| High MW guar gum (g) | 3.0 | 3.0 | 3.0 | - | - | - |
| Low MW guar gum (g) | - | - | - | 4.3 | 4.3 | 4.3 |
| Collagen (g) | - | 10 | 15 | - | 10 | 15 |
| Water (g) | 197 | 187 | 182 | 195.7 | 185.7 | 180.7 |
| Viscosity (mPa.s) | 30600 | 110 | 19 | 31600 | 240 | 35 |

### C. Viscosity lowering effect of different collagen concentration on the viscosity of a 1.5% by weight, guar gum composition (high MW type).

| | | | | | | |
|---|---|---|---|---|---|---|
| Guar gum (g) | 3 | 3 | 3 | 3 | 3 | 3 |
| Collagen (g) | 0 | 2.5 | 5 | 7.5 | 10 | 15 |
| Water (g) | 197 | 194.5 | 192 | 189.5 | 187 | 182 |
| Viscosity (mPa.s) | 31000 | 26000 | 5300 | 110 | 67 | 29 |

### Example 5: (Comparative Example) Viscosity of xanthan gum compositions.

The viscous soluble fiber compositions were prepared as described in Example 1, and the viscosities were measured with a Brookfield, spindle LV DV at 20°C.

### A. Influence of collagen concentration on the viscosity of a 1% by weight xanthan gum composition.

| | | | | | | |
|---|---|---|---|---|---|---|
| Xanthan gum (g) | 2 | 2 | 2 | 2 | 2 | 2 |
| Collagen (g) | 0 | 2.5 | 5 | 7.5 | 10 | 15 |
| Water (g) | 198 | 195.5 | 193 | 190.5 | 188 | 183 |
| Viscosity (mPa.s) | 760 | 520 | 3050 | 6300 | 30400 | 41000 |

### B. Influence of collagen on the viscosity of xanthan gum compositions.

| | | | | | | |
|---|---|---|---|---|---|---|
| Xanthan gum (g) | 0.4 | 0.4 | 1 | 1 | 2 | 2 |
| Collagen(g) | - | 10 | - | 10 | - | 10 |
| Water (g) | 199.6 | 189.6 | 199 | 189 | 198 | 188 |
| Viscosity (mPa.s) | 120 | 15 | 300 | 2500 | 760 | 30400 |

### C. Influence of the acidity on the viscosity of a 0.5% by weight xanthan gum composition.

| | | | | |
|---|---|---|---|---|
| Xanthan gum (g) | 1 | 1 | 1 | 1 |
| Collagen (g) | 5 | 10 | 5 | 10 |
| Citric acid (g) | - | - | 0.05 | 0.05 |
| Water (g) | 194 | 189 | 194 | 189 |
| pH | 5.55 | 5.55 | 5.15 | 5.15 |
| Viscosity (mPa.s) | 1500 | 570 | 280 | 20 |

### Example 6: (Comparative Example) Interactions between guar gum and xanthan gum.

The compositions were prepared as in Example 1.The viscosity was measured with a Brookfield viscosimeter (spindle LV) at 20°C.

### A. Effects on the viscosity.

| | | | | | |
|---|---|---|---|---|---|
| Xanthan gum(g) | 0 | 0.3 | 0.9 | 1.5 | 2.1 |
| Guar gum (g) | 3 | 2.7 | 2.1 | 1.5 | 0.9 |
| Water (g) | 197 | 197 | 197 | 197 | 197 |
| Viscosity (mPa.s) | 49000 | 212000 | 60000 | 6000 | 2100 |

### B. Influence of collagen 5% by weight on the viscosity.

| | | | | | |
|---|---|---|---|---|---|
| Xanthan gum (g) | 0 | 0.3 | 0.9 | 1.5 | 2.1 |
| Guar gum(g) | 3 | 2.7 | 2.1 | 1.5 | 0.9 |
| Collagen(g) | 10 | 10 | 10 | 10 | 10 |
| Water (g) | 187 | 187 | 187 | 187 | 187 |
| Viscosity (mPa.s) | 100 | 15000 | 98000 | 200000 | 300000 |

### C. Influence of acidity and collagen 5% by weight on the viscosity.

| | | | | | |
|---|---|---|---|---|---|
| Xanthan gum(g) | 0 | 0.3 | 0.9 | 1.5 | 2.1 |
| Guar gum (g) | 3 | 2.7 | 2.1 | 1.5 | 0.9 |
| Collagen(g) | 10 | 10 | 10 | 10 | 10 |
| Citric acid (g) | 0.05 | 0.05 | 0.05 | 0.05 | 0.05 |
| Water(g) | 187 | 187 | 187 | 187 | 187 |
| Viscosity (mPa.s) | 100 | 1200 | 200000 | 320000 | 340000 |

### D. Influence of acidity and collagen 2.5% by weight on the viscosity.

| | | | | | | | |
|---|---|---|---|---|---|---|---|
| Xanthan gum (g) | 0 | 0 | 0.3 | 0.3 | 0.9 | 0.9 | 0.9 |
| Guar gum (g) | 3 | 3 | 2.7 | 2.7 | 2.1 | 2.1 | 2.1 |
| Collagen (g) | 5 | 5 | 5 | 5 | 5 | 5 | 5 |
| Citric acid (g) | 0.025 | 0.25 | 0.025 | 0.25 | 0.025 | 0.25 | 1 |
| Water (g) | 198 | 198 | 198 | 198 | 198 | 198 | 197 |
| Viscosity (mPa.s) | 100 | 5000 | 1200 | 25000 | 200000 | 42600 | 39000 |

### Example 7: (Comparative Example) : Preparation of a lemon instant drink containing guar gum and collagen.

The different ingredients are admixed together and mixed with 264.4 g of water at temperature of about 25°C, in a shaker, for about 10 seconds.

| | |
|---|---|
| Guar gum⁽¹⁾ (g) | 3.75 |
| Collagen (g) | 17.3 |
| Fructose (g) | 12.3 |
| Na₂Hpyrophosphate (g) | 0.9 |
| Citric acid (g) | 0.5 |
| Lemon flavor (g) | 0.7 |
| Aspartame⁽²⁾ (g) | 0.015 |
| Acesulfam K ⁽³⁾ (g) | 0.015 |
| Acetic ester of mono | |
| and diglycerides (g) | 0.08 |

| | |
|---|---|
| ⁽¹⁾ The guar gum is of high molecular weight type. | |

The final composition has a pH of 4.35 and a viscosity between about 25 and about 30 mPa.s.

## Claims

1. Use of collagen hydrolysate to lower the viscosity of a composition comprising one or more viscous soluble fiber(s) wherein said viscous soluble fiber is guar gum.

2. Use of a composition comprising one or more viscous soluble fiber(s) and collagen hydrolysate having a viscosity of less than 500 mPa.s at room temperature, wherein said viscous soluble fiber is guar gum for the manufacture of a medicament for the prevention or treatment of metabolic syndrome and/or diabetes and/or for lowering blood cholesterol level.

3. The use of claim 2, wherein the weight ratio of said fiber: said protein is from 0.01:1 to 20:1.

4. The use of claim 2 or 3, wherein the composition comprises 1% to 10% by weight of viscous soluble fibers, based on the total weight of the composition.

5. A composition comprising one or more viscous soluble fiber(s) and collagen hydrolysate having a viscosity of less than 500 mPa.s at room temperature, wherein said viscous soluble fiber is guar gum for use in the prevention or treatment of metabolic syndrome and/or diabetes and/or for lowering blood cholesterol level.

6. The composition for use of claim 5, wherein the weight ratio of said fiber: said protein is from 0.01:1 to 20:1.

7. The composition for use of claim 5 or 6, wherein the composition comprises 1% to 10% by weight of viscous soluble fibers, based on the total weight of the composition.

8. The composition for use of any one of claims 5 to 7 wherein the composition is in the form of a drink.

## Patentansprüche

1. Verwendung von Collagen-Hydrolysat zur Verringerung der Viskosität einer Zusammensetzung, die eine oder mehrere viskose, lösliche Faser(n) aufweist, wobei die viskose, lösliche Faser Guargummi ist.

2. Verwendung einer Zusammensetzung, die eine oder mehrere viskose, lösliche Faser(n) und Collagen-Hydrolysat mit einer Viskosität von weniger als 500 mPas bei Raumtemperatur aufweist, wobei die viskose, lösliche Faser Guargummi ist, zur Herstellung eines Medikaments zum Schutz vor oder zur Behandlung des metabolischen Syndroms und/oder von Diabetes und/oder zum Herabsetzen des Blutcholesterinspiegels.

3. Verwendung gemäß Anspruch 2, wobei das Gewichtsverhältnis der Faser zu dem Protein von 0,01:1 bis 20:1 beträgt.

4. Verwendung gemäß Anspruch 2 oder 3, wobei die Zusammensetzung 1 Gew.-% bis 10 Gew.-% viskose, lösliche Fasern basierend auf dem Gesamtgewicht der Zusammensetzung, aufweist.

5. Zusammensetzung, die eine oder mehrere viskose, lösliche Faser(n) und Collagen-Hydrolysat mit einer Viskosität von weniger als 500 mPa:s bei Raumtemperatur aufweist, wobei die viskose, lösliche Faser Guargummi ist, für die Verwendung zum Schutz vor oder zur Behandlung des metabolischen Syndroms und/oder von Diabetes und/oder zum Herabsetzen des Blutcholesterinspiegels.

6. Zusammensetzung zur Verwendung gemäß Anspruch 5, wobei das Gewichtsverhältnis der Faser zu dem Protein von 0,01:1 bis 20:1 beträgt.

7. Zusammensetzung zur Verwendung gemäß Anspruch 5 oder 6, wobei die Zusammensetzung 1 Gew.-% bis 10 Gew.-% viskose, lösliche Fasern, basierend auf dem Gesamtgewicht der Zusammensetzung enthält.

8. Zusammensetzung zur Verwendung gemäß irgendeinem der Ansprüche 5 bis 7, wobei die Zusammensetzung in der Form eines Getränks vorliegt.

## Revendications

1. Utilisation d'hydrolysat de collagène pour réduire la viscosité d'une composition comprenant une ou plusieurs fibre(s) soluble(s) visqueuse(s), ladite fibre soluble visqueuse étant de la gomme de guar.

2. Utilisation d'une composition comprenant une ou plusieurs fibre(s) sollible(s) visqueuse(s) et de l'hydrolysat de collagène ayant une viscosité de moins de 500 mPa.s à température ambiante,
ladite fibre soluble visqueuse étant de la gomme de guar pour la fabrication d'une médicament pour la prévention ou le traitement de syndrome métabolique et/ou diabètes et/ou pour diminuer niveau de cholestérol dans le sang.

3. Utilisation selon la revendication 2,
le rapport de poids de ladite fibre : ladite protéine étant compris entre 0,01 : 1 et 20:1.

4. Utilisation selon la revendication 2 ou 3,
la composition comprenant 1% à 10 % en poids de fibres solubles visqueuses, par rapport au poids total de la composition.

5. Composition comprenant une ou plusieurs fibre(s) soluble(s) visqueuse(s) et du hydrolysat de collagène ayant une viscosité de moins de 500 mPa.s à température ambiante,
ladite fibre soluble visqueuse, étant de la gomme de glua, étant pour l'utilisation dans la fabrication d'une médicament pour la prévention ou le traitement de syndrome métabolique et/ou diabètes et/ou pour diminuer le niveau de cholestérol dans le sang.

6. Composition pour utilisation selon la revendication 5,
le rapport de poids de ladite fibre : ladite protéine étant compris entre 0,01:1 et 20 : 1.

7. Composition pour utilisation selon la revendication 5 ou 6,
la composition comprenant 1 % à 10 % en poids de fibres solubles visqueuses, par rapport au poids total de la composition.

8. Composition pour utilisation selon l'une des revendications 5 à 7,
la composition étant de la forme d'une boisson.
